# EUROPEAN PATENT SPECIFICATION

(11) **EP 3 900 751 B1**
(45) Date of publication and mention of the grant of the patent: **20.08.2025**
(21) Application number: 19900506.7
(22) Date of filing: 19.12.2019
(51) Int. Cl.: A61L 27/20, A61L 27/52, A61L 27/50, A61L 27/54, A61F 2/00

(54) **FILLER HAVING EXCELLENT FILLER PROPERTIES COMPRISING HYALURONIC ACID HYDROGEL**
FÜLLSTOFF MIT HERVORRAGENDEN FÜLLSTOFFEIGENSCHAFTEN UND MIT HYALURONSÄUREHYDROGEL
CHARGE PRÉSENTANT D'EXCELLENTES PROPRIÉTÉS DE CHARGE COMPRENANT UN HYDROGEL D'ACIDE HYALURONIQUE

(30) Priority: 20.12.2018 KR 20180166747
(43) Date of publication of application: 27.10.2021
(73) Proprietor: LG Chem, Ltd., Seoul 07336, (KR)
(72) Inventor: JANG, Cheol, Daejeon 34122 (KR); LEE, Chung, Daejeon 34122 (KR); KIM, Ji Sun, Daejeon 34122 (KR); JUNG, Hyun Tae, Daejeon 34122 (KR); LEE, Chang Hyun, Daejeon 34122 (KR); SO, Jineon, Daejeon 34122 (KR); REE, Hwayoun, Daejeon 34122 (KR)
(74) Representative: Goddar, Heinz J.
(86) International application number: PCT/KR2019/018128
(87) International publication number: WO 2020/130684

(56) References cited:
- EP-A1- 3 804 769
- KR-A- 20120 006 451
- KR-A- 20150 029 578
- KR-A- 20170 027 090
- KR-A- 20170 118 105
- KR-B1- 101 660 211

## Description

### [TECHNICAL FIELD]

### CROSS-REFERENCE TO RELATED APPLICATION(S)

This application claims the benefit of priority based on Korean Patent Application No. 10-2018-0166747 filed on December 20, 2018 with the Korean Intellectual Property Office.

The present invention relates to a filler containing a hyaluronic acid hydrogel, and more particularly to a filler containing a hyaluronic acid hydrogel wherein a ratio of lift capability of an unit content (w/w%) of hyaluronic acid contained in the filler relative to a modification degree (MoD) is high and thus, the filler exhibits excellent properties and has excellent wrinkle improvement and tissue restoration capacity, and a method for preparing the same.

### [BACKGROUND ART]

The tissue of the human skin maintains its structure through an extracellular matrix containing proteins such as collagen or elastin, etc., and glycosaminoglycans. When a soft tissue defect occurs by external shock, diseases or aging or the like, tissue enhancement such as soft tissue enhancement has been used for medical and cosmetic purposes. Such enhancement has been made surgically via plastic surgery, or the shape has been restored and corrected in a non-surgical manner by injecting biological tissues or synthetic polymer chemicals into an affected site to increase and expand the volume of soft tissue. In this case, a material which is inserted as a component similar to a skin tissue into a specific site to augment soft tissue and thereby enlarge the volume of cheeks, lips, breast, hips, or the like for cosmetic purposes, and which is used for wrinkle improvement or contour correction by reducing fine wrinkles and deep wrinkles on the skin, is referred to as a material for soft tissue augmentation and also generally referred to as a dermal filler. The first-generation dermal filler developed for the first time in connection with these fillers includes products such as Zyderm and Zyplast produced by extracting animal-derived proteins, that is, animal proteins such as cows and pigs, and Cosmoderm or Cosmoplast produced using human collagen. However, it is rarely used for surgical operation in recent years because of a short duration of pharmaceutical effect and an inconvenience that a skin hypersensitivity test must perform one month before the operation.

The second-generation filer is hyaluronic acid (also referred to as 'HA') filler, which has a longer duration of effect than a collagen filler and is composed of N-acetyl-D-glucosamine and D-glucuronic acid, which are polysaccharides similar to the elements that make up the human body. Accordingly, it has the advantage that it has few side effects such as skin hypersensitivity reaction or the like, is easy to operate and remove, and can attract water to maintain skin moisture and also maintain the volume and elasticity and thus is suitable as a skin filler.

However, hyaluronic acid itself has a short half-life of only a few hours in the human body, and its application is limited, and therefore, studies have been conducted to increase the half-life (persistence in the body) of hyaluronic acid via crosslinking. For example, U.S. Patent No. 4,582,865 discloses a crosslinked hyaluronic acid derivative using divinyl sulfone (DVS) as a crosslinking agent, and the hydrogel form thereof has been marketed under the trade name of Hylaform^{®}. In addition, U.S. Patent No. 5,827,937 discloses a method for preparing crosslinked hyaluronic acid derivatives by using a polyfunctional epoxy compound as a crosslinking agent, and among them, Restylane^{®}, a crosslinked hyaluronic acid in the form of a hydrogel prepared using 1,4-butanediol diglycidyl ether (BDDE) (crosslinking agent) as a polyfunctional epoxy compound, is approved by the U.S Food and Drug Administration (FDA) and commercially available worldwide as a filler.

Such crosslinked hyaluronic acid filler includes a filler made of a single phase (monophasic HA filler) and a filler made of a bi-phase (biphasic HA filler). Since monophasic hyaluronic acid filler is prepared using a homogeneous solution containing crosslinked hyaluronic acid, it has low elasticity and high cohesivity. Thus, when the monophasic hyaluronic acid filler is injected into the skin, it is unlikely to detach from the injected site, but has a problem that the injected shape cannot be maintained for a long time.

Biphasic hyaluronic acid fillers are prepared from crosslinked hyaluronic acid particles alone or prepared by mixing with a non-crosslinked hyaluronic acid (non-treated, non-crosslinked hyaluronic acid, linear HA) similar to a liquid phase, and thus, they generally have high elasticity and low cohesivity. Accordingly, when the biphasic HA fillers are injected into the skin, the shape can be maintained for a long time, but there is a problem that the likelihood of detachment from the injected site is high. A typical example of such a biphasic HA filler is Restylane^{®} (Galderma) mentioned above.

As such, the monophasic HA fillers and the biphasic HA fillers have advantages and disadvantages, respectively, and, there is an example in which the fillers are mixed so as to have all the properties of the conventional monophasic hyaluronic acid filler and biphasic hyaluronic acid filler, but in such a case, the advantages of the monophasic hyaluronic acid filler and the biphasic hyaluronic acid filler are rather reduced together, making them unsuitable as fillers. Therefore, there is a need for a filler capable of maintaining its shape for a long time while having a low possibility of detaching the injected site.

D2 KR 2015 0029578 A discloses fillers comprising x-linked hyaluronic acid.

Furthermore, in order to adjust the rheological properties such as elasticity and cohesivity of the crosslinked hyaluronic acid and the degradation period in this way, the concentration of the hyaluronic acid reacted during the preparation of the crosslinked product and the ratio of the crosslinking agent added must be adjusted from the beginning. Further, in general, in order to improve the rheological properties or the degradation period, it is necessary to increase the reaction concentration or add a large amount of a crosslinking agent. In this case, it is not easy to produce at a high concentration, and it is difficult to completely remove the crosslinking agent added in a large amount during the purification process, which may cause problems such as toxicity due to the remaining crosslinking agent.

### [DETAILED DESCRIPTION OF THE INVENTION]

### [Technical Problem]

The present invention has been designed to solve the problems encountered in the prior art, and it is an object of the present invention to provide a filler containing a hyaluronic acid hydrogel which has excellent properties in both elasticity and cohesivity that are indicators of the performance of the filler, makes it less likely to detach from the injected site, can keep the shape for a long time, and has low content of crosslinking agent while having excellent effects such as tissue restoration capacity and wrinkle improvement, and thus, exhibits excellent biocompatibility, and a biomaterial for tissue repair containing the aforementioned hyaluronic acid hydrogel filler.

### [Technical Solution]

The present inventors have conducted research to solve the problems of the prior art as described above, and as a result, have found, unexpectedly, that when a ratio of lift capability of an unit content (w/w%) of hyaluronic acid contained in the filler relative to a modification degree (MoD) indicates a specific range, both elasticity and cohesivity are improved and thus, rheological properties of a high cohesivity of the monophasic filler and a high elasticity of the biphasic filler are achieved at the same time. Furthermore, when the ratio of lift capability of the unit content (w/w%) of hyaluronic acid contained in the filler relative to the modification degree indicates a specific range, the filler is easily made into a desired form when injecting into the human body, sustains the shape for a desired period, exhibits excellent filler properties that the filler component is not substantially migrated to other parts of the body, and can minimize toxic problems capable of occurring in the body due to reduced use of crosslinking agents, and furthermore, it is possible to reduce the likelihood of immune reactions and side effects by minimizing the modification of hyaluronic acid and maintaining the hyaluronic acid molecule in a natural state. The present invention has been completed on the basis of such findings.

The scope of the invention is defined by the claims. Any references in the description to methods of treatment refer to the products of the present invention for use in a method for treatment.

As one aspect for achieving the object of the invention described above, the present invention relates to a filler as defined in claim 1.

Preferably, the filler is for injection to soft tissue, such as skin, and the filler may be used as a filler having filling properties, for example, such as wrinkle improvement due to the filling of biological tissues and the filling of wrinkles, remodeling of the face or contouring , or repair or increase in the volume of soft tissue.

Hereinafter, the present invention will be described in more detail.

The hyaluronic acid (hereinafter, also referred to as 'HA') contained in the hyaluronic acid hydrogel including in the filler of the present invention is a biopolymer material in which disaccharide units composed of N-acetyl-D-glucosamine and D-glucuronic acid are linearly connected, and the hyaluronic acid is often present in a vitreous humor of the eye, a synovial fluid of joints, rooster comb, and the like, and has excellent biocompatibility, and thus, has been widely used in the medical care and medical instrument fields such as ophthalmic surgical aids, joint function improvers, drug delivery materials, instillations, wrinkle improvers and the like, or in the cosmetics field.

In a preferred embodiment, the filler containing hyaluronic acid hydrogel according to the present invention may include 1 to 3% by weight of hyaluronic acid based on the total weight of the filler.

Further, the filler containing the hyaluronic acid hydrogel according to the present invention may further include not only hyaluronic acid but also water, an anesthetic agent, or a combination thereof.

Specifically, the hyaluronic acid contained in the hyaluronic acid hydrogel according to the present invention may refer to hyaluronic acid or a salt of hyaluronic acid. The salt of hyaluronic acid includes, for example, both inorganic salts such as sodium hyaluronate, potassium hyaluronate, calcium hyaluronate, magnesium hyaluronate, zinc hyaluronate, cobalt hyaluronic acid, and the like, and organic salts such as hyaluronic acid tetrabutylammonium or the like, but is not limited thereto.

Further, the hyaluronic acid or a salt thereof is crosslinked by a suitable crosslinking agent. The crosslinked hyaluronic acid derivative may be a crosslinked product of hyaluronic acid or hyaluronic acid salt prepared by crosslinking the hyaluronic acid itself or a salt thereof using a crosslinking agent. The hyaluronic acid hydrogel according to the present invention includes a crosslinked product of hyaluronic acid. In the present invention, the crosslinking agent includes the crosslinking agent itself or a crosslinking agent in a form that is reacted with hyaluronic acid and bonded to the molecular of hyaluronic acid. The hyaluronic acid hydrogels according to the present invention exhibit low modification degree as mentioned below and include both crosslinked hyaluronic acid and non-crosslinked hyaluronic acid.

For the crosslinking, a method of using a crosslinking agent in the presence of an aqueous alkaline solution may be used. The aqueous alkaline solution may be NaOH and KOH, preferably NaOH aqueous solution, but is not limited thereto. In this case, the NaOH aqueous solution may be used at a concentration of 0.1 to 0.5 N. For the crosslinked hyaluronic acid contained in the filler of the present invention, in particular, even when the crosslinking agent is used in a low concentration and in a small amount, the ratio of lift capability of the unit content (w/w%) of hyaluronic acid contained in the filler relative to the modification degree (mol%) of the crosslinked hyaluronic acid shows a specific range and thus exhibits high rheological properties (elasticity, cohesivity).

The crosslinking agent may vary as a compound including two or more epoxy functional groups, and preferred examples thereof include 1,4-butanediol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy) ethylene, pentaerythritol polyglycidyl ether, and sorbitol polyglycidyl ether. Among them, biepoxide-based 1,4-butanediol diglycidyl ether is particularly preferred in terms of having low toxicity.

In the present invention, the average molecular weight of the hyaluronic acid used in the crosslinking reaction may be 2,000,000 Da or more, 2,300,000 Da or more, or 2,500,000 Da or more, for example, 2,000,000 to 4,000,000 Da, 2,300,000 to 4,000,000 Da, 2,000,000 to 3,700,000 Da, 2,200,000 to 3,700,000 Da, or 2,500,000 to 3,500,000 Da.

The term "lift capability" as used herein is also referred to as 'tissue restoration capacity', and when the physical property of the hydrogel is measured with a rheometer, and it can be expressed as a value obtained by multiplying a storage elastic modulus reflecting elasticity and a cohesive force at tack test reflecting cohesivity. Specifically, it is expressed as in Equation 1 below. Lift Capability (Pa * N) = Storage Elastic Modulus × Cohesive Force

In Equation 1, the storage elastic modulus (G') of the filler is the elasticity of the filler and the unit is Pascal (Pa), and the cohesive force is a peak normal force measured by tack test, which involved stretching the samples axially at a constant velocity, and the unit is N (newton).

In relation to the physical properties of the filler measured by the rheometer, the term "elasticity", indicated by the storage elastic modulus, refers to a property in which the shape changes when a force is applied to a material or an object but returns to its original shape when the force is removed. The elasticity is represented by a storage elastic modulus (G'), and the unit is Pascal (Pa). In a preferred embodiment, the hyaluronic acid hydrogel filler according to the present invention exhibits an elasticity having a storage elastic modulus of about 400 to 800 Pa.

Further, the term "cohesivity" of the filler is an attractive force (adhesive force) acting between filler particles, which means the property of allowing the filler particles to aggregate. As the cohesivity of the filler is higher, the force capable of supporting the tissue into which the filler is injected is larger. In general, the cohesivity can be measured by a tack test or the like, and the cohesive force under stretching at a constant speed after loading onto a rheometer is measured, and the unit is N (newton). The hyaluronic acid hydrogel filler according to the present invention exhibits a cohesivity of about 1.0 to 1.7 N.

The lift capability expressed as a product of elasticity and cohesivity which is measured by a rheometer is a capability of expanding or restoring the injected site of the filler. A high lift capability means that the tissue restoration capacity is excellent and the migration of the filler after injection of the filler is small.

In the present invention, the lift capability of the hyaluronic acid hydrogel or the filler including the same is used as a value obtained by multiplying the storage elastic modulus shown in Equation 1 and the cohesive force at tack test, or can be expressed as the lift capability with respect to the content (w/w%) of hyaluronic acid contained in the filler.

In the filler containing a hyaluronic acid hydrogel according to one embodiment of the present invention, the numerical value of the lift capability with respect to the unit content (w/w%) of hyaluronic acid contained in the filler may indicate a range of 200 (Pa*N/w/w%) or more, 230 (Pa*N/w/w%) or more, or 250 (Pa*N/w/w%) or more, for example, it may indicate a range of 200 to 650 (Pa*N/w/w%), 230 to 650(Pa*N/w/w%), 240 to 650(Pa*N/w/w%), 250 to 650(Pa*N/w/w%), 200 to 640(Pa*N/w/w%), 230 to 640(Pa*N/w/w%), 240 to 640(Pa*N/w/w%), 250 to 640(Pa*N/w/w%), 200 to 630(Pa*N/w/w%), 230 to 640(Pa*N/w/w%), 240 to 630(Pa*N/w/w%), or 250 to 630 (Pa*N/w/w%).

Meanwhile, in the present invention, the term "modification degree (MoD)" is a ratio (mol%) of the number of moles (n) of the crosslinking agent (e.g., BDDE) to the number of moles of the disaccharide unit (N-acetyl-D-glucosamine (GlcNAc) + D-glucuronic acid) which is a repeating unit of hyaluronic acid contained in the hyaluronic acid hydrogel obtained by subjecting hyaluronic acid to a crosslinking treatment, and is calculated at stoichiometric ratio. The modification degree (mol%) of the hyaluronic acid hydrogel may be represented by the following Equation 2. Modification Degree (mol%) = (The number of moles of the crosslinking agent/The number of moles of the (N- acetyl-D-glucosamine + D-glucuronic acid)) * 100

In the present invention, in particular, hyaluronic acid hydrogels in the filler containing commercially available hyaluronic acid hydrogels exhibit a modification degree of 6 mol% or more, whereas the hyaluronic acid hydrogel in the fillers according to the present invention indicates a modification degree range of 2 to 5 mol%, 3 to 5 mol%, 2 to 4 mol%, or 3 to 4 mol%.

Preferably, the filler containing the hyaluronic acid hydrogel according to the present invention has the feature that a value of the ratio of lift capability of the unit content (w/w%) of the hyaluronic acid contained in the filler relative to the modification degree of the hyaluronic acid hydrogel has the above range, and thus, the filler has excellent filler properties, specifically not only high cohesivity like monophasic fillers but also high elasticity like biphasic fillers, and further, the content of the crosslinking agent is minimized and the modification of hyaluronic acid is minimized, and it exhibits low immune response and potential side effects by maintaining hyaluronic acid molecules in a natural state to the maximum.

Specifically, in the filler according to the present invention, the ratio of lift capability of the unit content (w/w%) of the hyaluronic acid included in the filler relative to the modification degree of the filler is expressed by Equation 3 below. Lift Capability per Hyaluronic Acid Unit Content and Modification Degree = A/B

In Equation 3, A is a lift capability with respect to the unit content (w/w%) of hyaluronic acid contained in the filler, and B means the modification degree of the filler according to Equation 2.

In addition, the crosslinked hyaluronic acid particles, in the filler containing the hyaluronic acid hydrogel according to the present invention may exhibit various shapes. Further, the average diameter of such particles may be 300 to 400 µm.

In a preferred embodiment, the filler containing hyaluronic acid hydrogel according to the present invention may include 1 to 3% by weight, preferably 1.5 to 2.5% by weight of hyaluronic acid relative to the total weight of the filler. In addition, the filler containing the hyaluronic acid hydrogel according to the present invention may further include not only hyaluronic acid but also water, an anesthetic agent, or a combination thereof.

The anesthetic agent include one or more types of anesthetic agents known in the art, preferably topical anesthetic agents, and the concentration of one or more anesthetic agent is in an amount effective to mitigate pain experienced upon injection of the composition. Examples of the anesthetic agent can be selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethisoquin, dimethocaine, diperodon, dycyclonine, ecgonidine, ecgonine, ethyl chloride, etidocaine, beta-eucaine, euprocin, fenalcomine, fomocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octocaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof. In one embodiment, the anesthetic agent may be lidocaine, for example, in the form of lidocaine hydrochloride.

In the filler containing a hyaluronic acid hydrogel according to the present invention, the concentration of the anesthetic agent included in the filler may be about 0.1% to about 1.0% by weight based on the total weight of the filler, for example, about 0.2% to about 0.5% by weight of the composition. Preferably, it may be 0.3% by weight.

Further, the concentration of the anesthetic agent in the composition described herein can be therapeutically effective, which means that it is the concentration adequate to provide a therapeutic benefit in terms of convenience of surgical operation and patient compliance without inflicting harm to the patient.

The filler according to the present invention may further include a buffer, and any buffer may be used without limitation as long as it is used in the preparation of hyaluronic acid hydrogel. Preferred examples of such buffer include at least one buffer selected from the group consisting of citric acid, sodium hydrogen phosphate, sodium dihydrogen phosphate, acetic acid, diethyl barbituric acid, sodium acetate, tris (hydroxymethyl)methylamino)propanesulfonic acid) (TAPS), 2-bis(2-hydroxyethyl)amino)acetic acid (Bicine), tris (hydroxymethyl) ammonium methane (Tris), N-(2-hydroxy-1,1-bis (hydroxymethyl)ethyl)glycine (Tricine), 4-(2-hydroxyethyl)-1-piperazineethanesulfonic acid (HEPES), 2-[[1,3-dihydroxy-2-(hydroxymethyl)propan-2-yl]amino]methanesulfonic acid (TES), and piperazine-N,N'-bis(2-ethanesulfonic acid) (PIPES), but is not limited thereto. The content of the components included in the buffer solution may be appropriately adjusted, and may be preferably contained at a concentration of 0.3 to 2.0 g/L relative to the buffer.

Further, the filler according to the present invention may further include an isotonic agent, and such an isotonic agent may be used without limitation as long as it is used for the preparation of hyaluronic acid hydrogel, and may be included in a buffer. As the preferred isotonic agent, sodium chloride may be used, but the isotonic agent is not limited thereto. The content of the isotonic agent may be appropriately adjusted as necessary, and may be contained in an amount of, for example, 7.0 to 9.0 g/L relative to the buffer, but is not limited thereto.

In one embodiment according to the present invention, a buffer containing sodium chloride, sodium hydrogen phosphate, and sodium dihydrogen phosphate in water for injection are used.

In an additional aspect, the filler containing the hyaluronic acid hydrogel according to the present invention may further include acceptable components which can be included in the preparation of the filler, in addition to the aforementioned components.

Furthermore, the present invention is characterized in that the residual crosslinking agent is not substantially included in the hyaluronic acid filler having a high parameter value according to Equation 3, and the residual crosslinking agent is preferably contained in an amount of 0.5 ppm or less, which is the limit of detection.

The filler containing the hyaluronic acid hydrogel according to the present invention exhibits excellent properties in terms of elastic properties, cohesivity, biocompatibility and toxicity by having the characteristic parameter according to Equation 3 in the range of 60 to 180, and thus, can be very effectively used for cosmetic or therapeutic purposes. As a specific example, the filler containing the hyaluronic acid hydrogel may be used for wrinkle improvement due to the filling of biological tissues and the filling of wrinkles, remodeling of the face, or repairing or increasing the volume of soft tissues such as lips, nose, hips, cheeks or breasts. The filler containing hyaluronic acid hydrogel may be administered in a dosage form suitable for such purposes, and may preferably be an injection.

A method to prepare a filler according to the present invention is a method including the steps of:
(a) putting hyaluronic acid or a salt thereof into a crosslinking agent and an aqueous alkaline solution, stirring and then reacting the mixture to prepare a crosslinked hyaluronic acid hydrogel;
(b) roughly cutting the hyaluronic acid hydrogel prepared in step (a);
(c) preparing a buffer solution;
(d) washing and swelling the cut hyaluronic acid hydrogel prepared in step (b) using the buffer solution prepared in step (c);
(e) crushing the hyaluronic acid hydrogel washed and swollen in step (d); and
(f) filling the hydrogel prepared in step (e) into a syringe and then sterilizing it.

Step (a) is a step of crosslinking a hyaluronic acid or a salt thereof using a crosslinking agent in an aqueous alkaline solution phase to prepare a crosslinked hyaluronic acid hydrogel, and with respect to the matters relating to a hyaluronic acid or salts thereof, a crosslinking agent, and a crosslinked hyaluronic acid hydrogel, those mentioned in the filler including the hyaluronic acid hydrogel can be similarly applied.

In particular, the method for preparing a filler containing a hyaluronic acid hydrogel according to the present invention is characterized by using a polymer hyaluronic acid having an average molecular weight of 2,000,000 Da or more, 2,300,000 Da or more, or 2,500,000 Da or more, for example, 2,000,000 to 4,000,000 Da, 2,300,000 to 4,000,000 Da, 2,000,000 to 3,700,000 Da, 2,200,000 to 3,700,000 Da, or 2,500,000 to 3,500,000 Da. Further, the aqueous alkaline solution can be used without limitation as long as it is known as an aqueous alkaline solution suitable for the preparation of hyaluronic acid hydrogel. For example, it may be NaOH, KOH, NaHCO₃, LiOH or a combination thereof, preferably NaOH. The concentration of the aqueous alkaline solution may be 0.1 to 0.5N, but is not limited thereto. When using hyaluronic acid in the molecular weight range as described above, the effect of degradation in the aqueous alkaline solution for crosslinking is relatively small, and thus, crosslinked hyaluronic acid gel having strong physical properties can be prepared.

Further, the method for preparing a filler containing a hyaluronic acid hydrogel according to the present invention is characterized in that hyaluronic acid or a salt thereof reacts at a high concentration. Specifically, the concentration of hyaluronic acid or a salt thereof is 10 to 25% by weight based on the total weight of the mixture of the aqueous alkaline solution, and the concentration of the crosslinking agent is 1 to 10 mol% relative to the unit of the above added hyaluronic acid or salt thereof. When the concentration of the crosslinking agent is used at a high concentration exceeding the above range, filler with excessively high elasticity is obtained. When the concentration is less than the above range, the elasticity is excessively low, thus being unable to exhibit proper viscoelasticity. Specifically, step (a) may be performed by mixing hyaluronic acid or a salt thereof with a crosslinking agent and an aqueous alkaline solution, stirring and homogeneously mixing the mixture. The crosslinking reaction can be performed at room temperature or higher, preferably at a temperature ranging from 25 to 40°C for 15 to 22 hours.

As for the cutting step, various known cutting steps of hyaluronic acid hydrogel can be used. In one embodiment, the crosslinked gel prepared after the reaction can be obtained in the shape of a cake, and this can be divided into half-moon shapes using a cutter such as straw cutter, for example, and divided into six. Thereafter, the cutting process can be performed by passing the gel divided as described above (preferably two or more times) using a rough cutting machine having a constant interval of the blade edges.

Step (c) is a step of preparing a buffer solution used to wash and swell the crosslinked hyaluronic acid hydrogel cut in step (b), and the buffer solution can be prepared according to the method for preparing a known buffer solution. In addition, the buffer solution may further include an anesthetic agent. In one specific embodiment of the present invention, the buffer solution was prepared by dissolving sodium monohydrogen phosphate (12 hydrate), sodium dihydrogen phosphate monohydrate (monohydrate), sodium chloride, and lidocaine hydrochloride in a buffer tank containing water for injection. The buffer solution may be used without limitation as long as it is used for the preparation of hyaluronic acid hydrogel. Preferred examples of such buffer solution include at least one buffer solution selected from the group consisting of citric acid, sodium hydrogen phosphate, sodium dihydrogen phosphate, acetic acid, diethyl barbituric acid, sodium acetate, tris(hydroxymethyl)methylamino)propanesulfonic acid) (TAPS), 2-bis(2-hydroxyethyl)amino)acetic acid (Bicine), tris(hydroxymethyl)ammonium methane (Tris), N-(2-hydroxy-1,1-bis (hydroxymethyl) ethyl) glycine (Tricine), 4-(2-hydroxyethyl)-1-piperazineethanesulfonic acid (HEPES), 2-[[1,3-dihydroxy-2-(hydroxymethyl)propan-2-yl]amino]methanesulfonic acid (TES), and piperazine-N,N'-bis(2-ethanesulfonic acid) (PIPES), but is not limited thereto.

Step (d) is a step of washing and swelling the crosslinked hyaluronic acid hydrogel cut in step (b) with the buffer solution prepared in step (c), and this step (d) may be repeated one or more times. When the washing and swelling are completed, the washing liquor can be removed.

Step (e) is a step of crushing the washed and swollen hydrogel, and this crushing may be performed by various crushing methods, but is preferably extrusion.

In an additional embodiment, the hydrogel prepared after step (e) may be subjected to processes such as sterilization and/or defoaming. It can be quantitatively filled, sealed and sterilized in a suitable container such as a syringe.

### [ADVANTAGEOUS EFFECTS]

Since the filler according to the present invention has a numerical characteristic of the lift capability of the unit content (w/w%) of the hyaluronic acid contained in the filler relative to the specific modification degree, it exhibits improved high viscoelastic properties, has the advantages of monophasic hyaluronic acid hydrogel fillers and biphasic hyaluronic acid hydrogel fillers together, and thus exhibits high tissue restoration properties. In addition, when injected into the skin, not only it maintains its shape for a long time while having a low possibility of migration, but also it can minimize the modification of hyaluronic acid due to the use of a minimal crosslinking agent and can maintain the hyaluronic acid molecule in a natural state and reduce the immune response and the possibility of side effects, and is excellent in restoration or volume expansion and wrinkle-improving properties of soft tissue such as cheek, breast, nose, lips, and hips.

### [DETAILED DESCRIPTION OF THE EMBODIMENTS]

Hereinafter, the present invention will be described in more detail by way of Examples. However, these Examples are given for illustrative purposes only, and the examples do not fall under the scope of the claims.

### [EXAMPLE]

### Example 1: Preparation 1 of Filler Containing Hyaluronic Acid Hydrogel

Sodium hyaluronate having a molecular weight of 2.5 MDa to 3.5 MDa, sodium hydroxide, and 1,4-butanediol diglycidyl ether (BDDE) were weighed. During the reaction, the concentration of sodium hyaluronate was 16 wt%, and the mol% of BDDE was 4% relative to the unit of sodium hyaluronate added. Separately, a sodium hydroxide (NaOH) aqueous solution at a concentration of 0.25 N was prepared and filtered. The weighed sodium hyaluronate, 0.25N sodium hydroxide aqueous solution, and 1,4-butanediol diglycidyl ether (BDDE) were put in a mixer tank and mixed homogeneously, and the mixer tank was placed in a constant temperature water bath and reacted overnight at a temperature of 30°C to complete a crosslinking reaction. Thereafter, the crosslinked hyaluronic acid hydrogel after the reaction was roughly cut. Meanwhile, salts and anesthetic agents were dissolved at a concentration of 1.26 g/L of sodium hydrogen phosphate hydrate (dodecahydrate), 0.46 g/L of sodium dihydrogen phosphate monohydrate (monohydrate), 7 g/L of sodium chloride and 3 g/L of lidocaine hydrochloride in a buffer tank containing water for injection to prepare a buffer solution.

Some of the buffer solution was used as a primary buffer solution and transferred to a washing tank through a 0.22 *µ*m filter. The roughly cut hyaluronic acid hydrogel previously prepared was transferred to a washing tank containing a primary buffer solution and then stirred. The hyaluronic acid hydrogel was subjected to a primary washing and swelling, and then, when the swelling was completed, the washing solution was removed. Subsequently, the secondary buffer solution was transferred to a washing tank through a 0.22 µm filter and then stirred, and the hydrogel was subjected to a secondary washing and swelling, and then, when the washing and swelling were completed, the washing solution was removed. Thereafter, the tertiary buffer solution was transferred to a washing tank through a suitable 0.22 µm filter and then stirred, and the hyaluronic acid hydrogel was subjected to a third washing and swelling. Then, when the washing and swelling were completed, the washing solution was removed.

After completion of the third washing and swelling, it was confirmed whether the pH of the washing solution was in the neutral range, and the hyaluronic acid hydrogel subjected to the washing and swelling was crushed and then transferred to an extruder tank to measure a weight, the buffer solution was added so that the weigh of gel reaches a target weight, and a primary content correction was performed. When the primary content correction was completed, the hyaluronic acid hydrogel was crushed and transferred to the extruder tank. Thereafter, the crushed hyaluronic acid hydrogel was transferred to a heat treatment tank and homogenized, after which the content was measured, and the buffer solution was added thereto to perform a secondary content correction. The hyaluronic acid hydrogel after the secondary content correction was heat-treated at a temperature of 121°C or more for at least 1 minute, and degassing was performed by stirring the hyaluronic acid hydrogel under reduced pressure before loading into a syringe. Thereafter, the hyaluronic acid hydrogel was vacuum-filled to each syringe by a predetermined amount and stoppered with a rubber stopper at the same time. The filled syringe was steam sterilized for at least 8 minutes at a temperature of 121°C or higher in the final sterilizer.

### Example 2: Preparation 2 of Filler Containing Hyaluronic Acid Hydrogel

Sodium hyaluronate having a molecular weight of 2.5 MDa to 3.5 MDa, sodium hydroxide, and 1,4-butanediol diglycidyl ether (BDDE) were weighed. During the reaction, the concentration of sodium hyaluronate was 15 wt%, and the mol% of BDDE was 4% relative to the unit of sodium hyaluronate added. Separately, a sodium hydroxide (NaOH) aqueous solution at a concentration of 0.25 N was prepared and filtered. The weighed sodium hyaluronate, 0.25N sodium hydroxide aqueous solution, and 1,4-butanediol diglycidyl ether (BDDE) were put in a mixer tank and mixed homogeneously, and the mixer tank was placed in a constant temperature water bath and reacted overnight at a temperature of 30°C to complete a crosslinking reaction. Thereafter, the crosslinked hyaluronic acid hydrogel after the reaction was roughtly cut. Meanwhile, salts and anesthetic agents were dissolved at a concentration of 1.26 g/L of sodium hydrogen phosphate hydrate (dodecahydrate), 0.46 g/L of sodium dihydrogen phosphate monohydrate (monohydrate), 7 g/L of sodium chloride and 3 g/L of lidocaine hydrochloride in a buffer tank containing water for injection to prepare a buffer solution.

Some of the buffer solution was used as a primary buffer solution and transferred to a washing tank through a 0.22 *µ*m filter. The roughtly cut hyaluronic acid hydrogel previously prepared was transferred to a washing tank containing a primary buffer solution and then stirred. The hyaluronic acid hydrogel was subjected to a primary washing and swelling, and then, when the swelling was completed, the washing solution was removed. Subsequently, the secondary buffer solution was transferred to a washing tank through a 0.22 µm filter and then stirred, and the hydrogel was subjected to a secondary washing and swelling, and then, when the washing and swelling were completed, the washing solution was removed. Thereafter, the tertiary buffer solution was transferred to a washing tank through a suitable 0.22 µm filter and then stirred, and the hyaluronic acid hydrogel was subjected to a third washing and swelling. Then, when the washing and swelling were completed, the washing solution was removed.

After completion of the third washing and swelling, it was confirmed whether the pH of the washing solution was in the neutral range, and the hyaluronic acid hydrogel subjected to the washing and swelling was crushed and then transferred to an extruder tank to measure a weight, the buffer solution was added so that the weight of gel reaches a target weight, and a primary content correction was performed. When the primary content correction was completed, the hyaluronic acid hydrogel was crushed and transferred to the extruder tank. Thereafter, the crushed hyaluronic acid hydrogel was transferred to a heat treatment tank and homogenized, after which the content was measured, and the buffer solution was added thereto to perform a secondary content correction. The hyaluronic acid hydrogel after the secondary content correction was heat-treated at a temperature of 121°C or more for at least 1 minute, and degassing was performed by stirring the hyaluronic acid hydrogel under reduced pressure before loading into a syringe. Thereafter, the hyaluronic acid hydrogel was vacuum-filled to each syringe by a predetermined amount and stoppered with a rubber stopper at the same time. The filled syringe was steam sterilized for at least 10 minutes at a temperature of 121°C or higher in the final sterilizer.

### Experimental Example 1: Investigation of Parameters Based on Equation 3 of Filler Comprising Hyaluronic Acid Hydrogel

The rheological properties of the prepared Examples 1 and 2 were analyzed using a rheometer. For comparison =r=th the elastic properties of a commercially available filler formulation including hyaluronic acid hydrogel were also analyzed and compared. The commercially available filler formulations and analysis conditions as Comparative Examples 1 to 10 are as follows.

### <Comparative Examples 1 to 10>

Comparative Example 1: Juvederm Volift with Lidocaine
Comparative Example 2: Juvederm Voluma with Lidocaine
Comparative Example 3: Stylage M Lidocaine
Comparative Example 4: Stylage L Lidocaine
Comparative Example 5: Stylage XL Lidocaine
Comparative Example 6: Teosyal PureSense Deepline Lidocaine
Comparative Example 7: Teosyal PureSense Ultradeep Lidocaine
Comparative Example 8: Teosyal PureSense Ultimate Lidocaine
Comparative Example 9: Belotero Intense Lidocaine
Comparative Example 10: Belotero Volume Lidocaine

### <Analysis Condition>

### Analysis condition of storage elastic modulus(G')

(1) Test instrument: Rheometer (Anton Paar Ltd., MCR301) Oscillatory and Rotational Rheometer
(2) Frequency: 1 Hz
(3) Temperature: 25 °C
(4) Strain: 4 %
(5) Measuring geometry: 25 mm plate/plate
(9) Measuring gap: 1.0 mm

### Analysis condition of cohesive force according to Tack test

(1) Test instrument: Rheometer (Anton Paar Ltd., MCR301) Oscillatory and Rotational Rheometer
(2) Gap: Initial position: 1.0 mm, Final position: 15 mm
(3) Speed: 0.93 mm/s
(4) Temperature: 25 °C
(5) Measuring geometry: 25 mm plate/plate

### Analysis condition of modification degree)

(1) Test instrument: FT-NMR System (Jeol Ltd., ECA500/ECZ400S),
(2) Pulse: 30°
(3) Scans: 512
(4) Relaxation time (delay): 5 s
(5) Temperature: 25 °C

Under the above analysis conditions, the storage elastic modulus (G') for each frequency, and the cohesivity measured by Tack test are shown in Table 1 below. In addition, the lift capability were calculated from the storage elastic modulus and the cohesivity based on Equation 1, and the modification degree was calculated according to Equation 2. Based on this, the lift capability of the unit content (w/w%) of the hyaluronic acid contained in the filler relative to the modification degree according to Equation 3 was calculated, and then the results of each numerical value are shown in Table 1 below.

**[Table 1]**

| Exampl e/Comp arativ e Exampl e | Present Invention | | Juvederm | | Stylage | | | Teosyal | | | Belotero | |
|---|---|---|---|---|---|---|---|---|---|---|---|---|
| | Examp le 1 | Exam ple 2 | Comparative Exam ple 1 | Comp arat ive Exam ple 2 | Comp arat ive Exam ple 3 | Compa rativ e Exam ple 4 | Compa rativ e Examp le 5 | Comp arat ive Exam ple 6 | Comp arat ive Exam ple 7 | Comp arat ive Exam ple 8 | Comp arat ive Exam ple 9 | Compa rativ e Examp le 10 |
| Storag e elasti c modulu s G' (Pa, at 1 Hz) | 747 | 448 | 314 | 310 | 191 | 225 | 264 | 249 | 374 | 409 | 149 | 280 |
| Cohesi vity based n Tack test(N ) | 1.57 | 1.17 | 0.65 | 0.88 | 1.05 | 1.54 | 1.80 | 1.31 | 1.47 | 1.20 | 1.34 | 1. 81 |
| Lift capabi lity (Pa*N) | 1173 | 524 | 203 | 272 | 201 | 347 | 475 | 326 | 550 | 491 | 200 | 507 |
| MoD (mol%) | 3.3 | 3.5 | 6.3 | 6 | 7.5 | 7.8 | 7.8 | 11.8 | 14 | 16.5 | 8.5 | 12.8 |
| HA Conten t (w/w) % | 2.0 | 2.1 | 1.75 | 2.0 | 1.6 | 2.40 | 2.6 | 2.5 | 2.5 | 2.2 | 2.55 | 2.6 |
| Lift capabi lity/H A conten t (Pa*N / (w/w) %) | 586.4 | 249. 6 | 115. 7 | 135. 8 | 125. 3 | 144.4 | 182.8 | 130. 5 | 219. 9 | 223. 1 | 78.3 | 194.9 |
| (Lift capab ility /HA conte nt) /M oD (Pa *N/ (w /w)%) /mol% ) | 177.7 | 71.3 | 18.4 | 22.6 | 16.7 | 18.5 | 23.4 | 11.1 | 15.7 | 13.5 | 9.2 | 15.2 |

As can be seen through Table 1, it was confirmed that the parameter according to Equation 3 of the filler containing a hyaluronic acid hydrogel according to examples 1 and 2 shows 71.3 and 177.7 Pa*N/(w/w)%)/mol%, whereas other commercially available fillers of Comparative Examples 1 to 9 show numerical values of 9.2 to 23.4 Pa *N/(w/w)%)/mol%). That is, it can be seen that the filler containing a hyaluronic acid hydrogel according to examples 1 and 2 shows the difference in the parameter values according to Equation 3 of at least 3 times (Example 2 compared to Comparative Example 5), up to 19 times (Example 1 compared to Comparative Example 9) as compared with the other commercially available fillers. It was confirmed that for Comparative Examples 1 to 10, numerous fillers are also not higher in the lift capability as compared with Examples 1 and 2, and that there are some comparative examples in which the lift capability show the same numerical values as in Example 2, but the numerical values of the modification degree are high, and thus, the actual parameters according to Equation 3 drop significantly.

Taken together, it could be confirmed that Examples 1 and 2 exhibit high parameter values according to Equation 3, that is, high lift capability of the unit content (w/w%) of hyaluronic acid contained in the filler relative to the modification degree. Thus, not only the filler exhibits significant physical properties but also it exhibits low modification degree. As can be seen from these, the use of a small amount of the crosslinking material can reduce the possibility of side effects which may occur due to the crosslinking agent during the injection of the filler in the body and thus is most suitable as a filler for injection into a human body.

## Claims

1. A filler comprising a hyaluronic acid hydrogel including
crosslinked hyaluronic acid,
wherein a ratio of lift capability of an unit content (w/w%) of the hyaluronic acid contained in the filler relative to a modification degree (mol%) represents 60 to 180 ((Pa*N/(w/w)%)/mol%) in accordance with Equation 3 below. Lift Capability per Hyaluronic Acid Unit Content and Modification Degree = A/B
wherein, A represents a value obtained by dividing a lift capability represented by Equation 1 below by the unit content *w/w%) of hyaluronic acid contained in the filler, and B represents a modification degree represented by Equation 2 below. Lift Capability (Pa*N) = Storage Elastic Modulus × Cohesive Force Modification degree (mol%) = (mole number of total crosslinking agent bonded to the entire hyaluronic acid molecule / the number of moles of total (N-acetyl-D-glucosamine + D- glucuronic acid)) * 100,
wherein the B value represents a modification degree of 2 to 5 mol%,
wherein the cohesive force represents 1.0 to 1.7 N,
wherein the Storage Elastic Modulus is measured with an Oscillatory and Rotational Rheometer at a frequency of 1 Hz, at 25 °C, with 4 % strain, a measuring gap of 25 mm plate/plate and a measuring gap of 1.0 mm, and
wherein the cohesive force is measured with an Oscillatory and Rotational Rheometer at an initial gap position of 1.0 mm and final gap position of 15 mm, with a speed of 0.93 mm/s, at 25 °C and a measuring geometry of 25 mm plate/plate,
wherein the modification degree is measured with a FT-NMR System, and
wherein Example 1 from EP patent application EP19830465.1, and Example 2 from EP patent application EP19830465.1 are not claimed.

2. The filler according to claim 1, wherein the A value represents 200 to 650 Pa*N/(w/w)%.

3. The filler according to claim 1, wherein the storage elastic modulus represents 400 to 800 Pa.

4. The filler according to claim 1, wherein the crosslinked hyaluronic acid is obtained by crosslinking with at least one crosslinking agent selected from the group consisting of 1,4-butanediol diglycidyl ether (BDDE), ethylene glycol diglycidyl ether (EGDGE), 1,6-hexanedioldiglycidyl ether, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, tri-methylpropane polyglycidyl ether, 1,2-(bis(2,3-epoxypropoxy)ethylene, pentaerythritol polyglycidyl ether, and sorbitol polyglycidyl ether, preferably 1,4-butanediol diglycidyl ether (BDDE).

5. The filler according to claim 4, wherein the crosslinking agent remains in an amount of 0.5 ppm or less.

6. The filler according to claim 1, further comprising an anesthetic agent, preferably lidocaine or a salt thereof.

7. The filler according to claim 6, wherein the filler is for skin injection, wrinkle improvement, soft tissue restoration or volume expansion, or contouring.

8. A prefilled syringe filled with the filler of any one of claims 1 to 7.

9. A biomaterial for tissue restoration comprising the filler of any one of claims 1 to 7.

## Patentansprüche

1. Füllstoff, umfassend ein Hyaluronsäure-Hydrogel, enthaltend
vernetzte Hyaluronsäure,
wobei ein Verhältnis der Hebefähigkeit eines Einheitsgehalts (w/w%) der in dem Füllstoff enthaltenen Hyaluronsäure relativ zu einem Modifikationsgrad (mol%) 60 bis 180 ((Pa*N/ (w/w)%)/mol%) gemäß nachstehender Gleichung 3 darstellt: Hebefähigkeit pro Hyaluronsäure-Einheitsgehalt und Modifikationsgrad = A/B
wobei A einen Wert darstellt, der durch Dividieren einer durch nachstehende Gleichung 1 dargestellten Hebefähigkeit durch den Einheitsgehalt *w/w%) der in dem Füllstoff enthaltenen Hyaluronsäure erhalten wird, und B einen durch nachstehende Gleichung 2 dargestellten Modifikationsgrad darstellt: Hebefähigkeit (Pa*N) = Speicherelastizitätsmodul × Kohäsionskraft Modifikationsgrad (mol%) = (Molzahl des gesamten Vernetzungsmittels, das an das gesamte Hyaluronsäuremolekül gebunden ist/Molzahl der gesamten (N-Acetyl-D-glucosamin + D- glucuronsäure)) * 100,
wobei der B-Wert einen Modifikationsgrad von 2 bis 5 mol% darstellt,
wobei die Kohäsionskraft 1,0 bis 1,7 N darstellt,
wobei der Speicherelastizitätsmodul mit einem Oszillations- und Rotationsrheometer bei einer Frequenz von 1 Hz, bei 25 °C, mit 4 % Dehnung, einem Messspalt von 25 mm Platte/Platte und einem Messspalt von 1,0 mm gemessen wird, und
wobei die Kohäsionskraft mit einem Oszillations- und Rotationsrheometer an einer Anfangsspaltposition von 1,0 mm und einer Endspaltposition von 15 mm, mit einer Geschwindigkeit von 0,93 mm/s, bei 25 °C und einer Messgeometrie von 25 mm Platte/Platte gemessen wird,
wobei der Modifikationsgrad mit einem FT-NMR-System gemessen wird, und
wobei Beispiel 1 aus der EP-Patentanmeldung EP19830465.1 und Beispiel 2 aus der EP-Patentanmeldung EP19830465.1 nicht beansprucht werden.

2. Füllstoff nach Anspruch 1, wobei der A-Wert 200 bis 650 Pa*N/(w/w)% darstellt.

3. Füllstoff nach Anspruch 1, wobei der Speicherelastizitätsmodul 400 bis 800 Pa darstellt.

4. Füllstoff nach Anspruch 1, wobei die vernetzte Hyaluronsäure durch Vernetzen mit mindestens einem Vernetzungsmittel, ausgewählt aus der Gruppe, bestehend aus 1,4-Butandioldiglycidylether (BDDE), Ethylenglykoldiglycidylether (EGDGE), 1,6-Hexandioldiglycidylether, Propylenglykoldiglycidylether, Polypropylenglykoldiglycidylether, Polytetramethylenglykoldiglycidylether, Neopentylglykoldiglycidylether, Polyglycerinpolyglycidylether, Diglycerinpolyglycidylether, Glycerinpolyglycidylether, Trimethylpropanpolyglycidylether, 1,2-(Bis(2,3-epoxypropoxy)ethylen, Pentaerythritpolyglycidylether und Sorbitolpolyglycidylether, vorzugsweise 1,4-Butandioldiglycidylether (BDDE), erhalten wird.

5. Füllstoff nach Anspruch 4, wobei das Vernetzungsmittel in einer Menge von 0,5 ppm oder weniger verbleibt.

6. Füllstoff nach Anspruch 1, ferner umfassend ein Anästhetikum, vorzugsweise Lidocain oder ein Salz davon.

7. Füllstoff nach Anspruch 6, wobei der Füllstoff für Hautinjektion, Faltenverbesserung, Weichgewebewiederherstellung oder Volumenexpansion oder Konturierung ist.

8. Vorgefüllte Spritze, die mit dem Füllstoff nach einem der Ansprüche 1 bis 7 gefüllt ist.

9. Biomaterial zur Gewebewiederherstellung, umfassend den Füllstoff nach einem der Ansprüche 1 bis 7.

## Revendications

1. Produit de comblement comprenant un hydrogel d'acide hyaluronique comprenant de l'acide hyaluronique réticulé,
dans lequel un rapport de la capacité de levage d'une teneur unitaire (w/w%) de l'acide hyaluronique contenu dans le produit de comblement par rapport à un degré de modification (mol%) représente 60 à 180 ((Pa*N/(w/w)%)/mol%) conformément à l'équation 3 ci-dessous. Capacité de levage par unité d'acide hyaluronique et degré de modification = A/B
A représente une valeur obtenue en divisant une capacité de levage représentée par l'équation 1 ci-dessous par le contenu unitaire *p/p%) d'acide hyaluronique contenu dans le produit de comblement, et B représente un degré de modification représenté par l'équation 2 ci-dessous. Capacité de levage (Pa*N) = Module d'élasticité de stockage × Force de cohésion Degré de modification (mol%) = (nombre de moles de l'agent de réticulation total lié à l'ensemble de la molécule d'acide hyaluronique/nombre de moles du total (N-acétyl-D-glucosamine + acide D-glucuronique)) * 100,
la valeur B représente un degré de modification de 2 à 5 mol%,
la force de cohésion représente 1,0 à 1,7 N,
le module d'élasticité de stockage est mesuré avec un rhéomètre oscillatoire et rotatif à une fréquence de 1 Hz, à 25 °C, avec une déformation de 4 %, un écart de mesure de 25 mm plaque/plaque et un écart de mesure de 1,0 mm, et
la force de cohésion est mesurée à l'aide d'un rhéomètre oscillatoire et rotatif à une position initiale de 1,0 mm et à une position finale de 15 mm, à une vitesse de 0,93 mm/s, à 25 °C et avec une géométrie de mesure de 25 mm plaque/plaque,
le degré de modification est mesuré à l'aide d'un système FT-NMR, et
dans lequel l'exemple 1 de la demande de brevet EP19830465.1 et l'exemple 2 de la demande de brevet EP19830465.1 ne sont pas revendiqués.

2. Produit de comblement selon la revendication 1, dans lequel la valeur A représente 200 à 650 Pa*N/(w/w)%.

3. Produit de comblement selon la revendication 1, dans lequel le module d'élasticité à l'entreposage représente 400 à 800 Pa.

4. Produit de comblement selon la revendication 1, dans lequel l'acide hyaluronique réticulé est obtenu par réticulation avec au moins un agent de réticulation choisi dans le groupe constitué de l'éther diglycidylique du butanediol 1,4 (BDDE), de l'éther diglycidylique de l'éthylène glycol (EGDGE), de l'éther diglycidylique de l'hexanediol 1,6, de l'éther diglycidylique du propylène glycol, de l'éther diglycidylique du polypropylène glycol, éther diglycidylique du polytétraméthylène glycol, éther diglycidylique du néopentyle glycol, éther polyglycidylique du polyglycérol, éther polyglycidylique du diglycérol, éther polyglycidylique du glycérol, éther polyglycidylique de triméthylpropane, éther polyglycidylique de 1,2-(bis(2,3-époxypropoxy)éthylène, pentaérythritol, et éther polyglycidylique de sorbitol, de préférence éther diglycidylique de 1,4-butanediol (BDDE).

5. Produit de comblement selon la revendication 4, dans lequel l'agent de réticulation reste en quantité inférieure ou égale à 0,5 ppm.

6. Produit de comblement selon la revendication 1, comprenant en outre un agent anesthésique, de préférence de la lidocaïne ou un de ses sels.

7. Produit de comblement selon la revendication 6, dans lequel le produit de comblement est destiné à l'injection cutanée, à l'amélioration des rides, à la restauration des tissus mous ou à l'expansion du volume, ou au modelage.

8. Seringue préremplie avec le produit de comblement de l'une des revendications 1 à 7.

9. Biomatériau pour la restauration tissulaire comprenant le produit de comblement de l'une des revendications 1 à 7.
